(19) Europäisches Patentamt / European Patent Office / Office européen des brevets

(11) **EP 3 208 196 B1**

(12) **FASCICULE DE BREVET EUROPEEN**

(45) Date de publication et mention
de la délivrance du brevet:
**31.10.2018 Bulletin 2018/44**

(51) Int Cl.:
***B64D 37/06*** *(2006.01)* ***G01F 23/292*** *(2006.01)*

(21) Numéro de dépôt: **17155527.9**

(22) Date de dépôt: **10.02.2017**

(54) **RESERVOIR DE CARBURANT PERFECTIONNE POUR AERONEF, ET DISPOSITIF DE DETECTION D'EAU**

PERFEKTIONIERTER KRAFTSTOFFTANK FÜR LUFTFAHRZEUG, UND DETEKTIONSVORRICHTUNG FÜR WASSER

IMPROVED FUEL TANK FOR AIRCRAFT, AND DEVICE FOR DETECTING WATER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.02.2016 FR 1651410**

(43) Date de publication de la demande:
**23.08.2017 Bulletin 2017/34**

(73) Titulaire: **Zodiac Aerotechnics**
**42230 Roche La Moliere (FR)**

(72) Inventeur: **REYNARD, Bruno**
**69340 FRANCHEVILLE (FR)**

(74) Mandataire: **Cabinet Laurent & Charras**
**3 place de l'Hotel de Ville**
**CS 70203**
**42005 Saint-Etienne Cedex 1 (FR)**

(56) Documents cités:
**EP-A1- 0 795 740      US-A- 5 399 876**
**US-A1- 2003 155 538**

## Description

## DOMAINE TECHNIQUE

La présente invention se rapporte au domaine technique des aéronefs et concerne un réservoir de carburant perfectionné pour aéronef, notamment pour avion, hélicoptère ou analogue, ainsi qu'un dispositif de détection d'eau, notamment dans le fond dudit réservoir de carburant.

## ART ANTERIEUR

Dans le domaine de l'aéronautique, il est relativement fréquent d'avoir de l'eau qui vient se stocker dans le fond des réservoirs de carburant des aéronefs. Cette eau est soit entrée dans le réservoir sous forme dissoute dans le carburant, soit liée à la condensation de l'air situé dans le réservoir et qui rentre lorsque l'aéronef change d'altitude, soit liée à des opérations de maintenance, ou à toute autre origine. La présence de cette eau nécessite la réalisation d'opérations de drainage desdits réservoirs.

Les opérations de drainage des réservoirs sont effectuées manuellement selon les préconisations de l'avionneur, à une fréquence prédéfinie, habituellement à intervalle de temps régulier de quelques vols ou de quelques jours. Les opérations de drainage présentent l'inconvénient de générer une immobilisation relativement longue de l'aéronef puisque, avant de fonctionner, il est nécessaire que le système de drainage dégèle après l'atterrissage de l'aéronef.

Ces opérations étant effectuées à des fréquences prédéfinies, ainsi il arrive parfois que des opérations de drainage non nécessaires soient réalisées, pour et lorsqu'il n'y a pas d'eau au fond des réservoirs ou que la quantité d'eau est relativement faible, entrainant des immobilisations de l'aéronef et des dépenses inutiles.

L'état actuel de la technique comprend le document EP 0795740 A1, qui montre un capteur d'eau pour réservoir de carburant d'aéronef et comprenant un élément optique à prisme et connecté à une unité de calcul et de traitement qui reçoit un signal dudit capteur et est apte à permettre d'identifier la présence d'air, de carburant ou d'eau ainsi que d'une interface eau/ carburant, et à afficher la présence d'air, d'eau et/ou de carburant en utilisant des indicateurs lumineux. Ce système nécessite toutefois un contrôle visuel fréquent par l'équipage de bord.

## EXPOSE DE L'INVENTION

L'un des buts de l'invention est donc de remédier à ces inconvénients en proposant un réservoir de carburant pour aéronef permettant d'éviter les immobilisations de l'aéronef et les dépenses inutiles liées aux opérations de drainage dudit réservoir, ou bien des contrôles fréquents par l'équipage de bord.

A cet effet, il a été mis au point un réservoir de carburant d'un aéronef comprenant un capteur d'eau, agencé au fond dudit réservoir et comprenant un élément optique apte à permettre de différencier l'eau du carburant connecté à des moyens de calcul et de traitement pour recevoir un signal de présence d'eau envoyé par ledit capteur, remarquable en ce que les moyens de calcul et de traitement sont configurés pour interpréter le signal de présence d'eau et fournir une information sur la nécessité d'effectuer ou non une opération de drainage en fonction de la quantité d'eau présente dans le fond du réservoir, et l'élément optique est connecté aux moyens de calcul et de traitement par l'intermédiaire d'au moins une fibre optique.

De cette manière, les moyens de calcul et de traitement sont aptes à indiquer, en fonction du signal envoyé par le capteur, s'il est nécessaire d'effectuer ou non une opération de drainage du réservoir. Les opérations de drainage inutiles ou des contrôles visuels fréquents sont donc évités, ce qui limite les immobilisations de l'aéronef et les dépenses inutiles.

De cette manière, la détection de l'eau est basée sur un phénomène optique qui est stable dans le temps et qui permet de s'affranchir de toute méthode de calcul prenant en compte le niveau de carburant présent dans le réservoir pour mesurer la quantité d'eau résiduelle. La mise en oeuvre d'au moins une fibre optique est avantageuse car elle permet de s'affranchir de la présence d'autres matériaux, tel que du cuivre par exemple. La sécurité du système est garantie.

Selon une forme de réalisation particulière, la fibre optique est connectée, d'une part, à l'élément optique et, d'autre part, à une source lumineuse pour envoyer au moins un rayon lumineux vers ledit élément optique, ledit élément optique étant apte :

- à réfléchir et à renvoyer le rayon lumineux dans la fibre optique vers les moyens de calcul et de traitement lorsque ledit élément optique est immergé dans de l'eau, et ;
- à réfracter et à ne pas renvoyer le rayon lumineux lorsque ledit élément optique est immergé dans du carburant.

L'invention est particulièrement avantageuse car elle utilise ainsi les propriétés physiques de l'eau et du carburant tel que l'indice de réfraction, qui est stable dans le temps, pour différencier l'eau du carburant. Aucune pièce mobile n'est mise en oeuvre de sorte que la maintenance du capteur est limitée, voire nulle.

Selon une forme de réalisation particulière, l'élément optique se présente sous la forme d'un prisme à section triangulaire isocèle de manière à définir au moins une face principale et deux faces convergentes. Le prisme est réalisé dans un matériau transparent à la lumière comprenant un indice de réfraction égal à n1, avec n1 supérieur à 1.33. La fibre optique est connectée orthogonalement à la face principale du prisme de sorte que le rayon lumineux incident est destiné à se réfracter et/ou se réfléchir sur une première face convergente, avec un angle il par rapport à la normale de la première face convergente. Le prisme est conçu de sorte que : $n1 \times \sin(i1) > 1.33$ qui est la valeur de l'indice de réfraction de l'eau.

Les caractéristiques du prisme sont calculées en application de la loi de Snell-Descartes qui indique que : $n1 \times \sin(i1) = n2 \times \sin(i2)$, avec n2 correspondant à l'indice de réfraction du milieu dans lequel est immergé le prisme, et i2 correspondant à l'angle du rayon réfracté par rapport à la normale de la première face convergente du prisme.

De cette manière, pour détecter la présence d'eau dans le fond du réservoir de carburant, il faut que le rayon incident soit totalement réfléchi par le prisme lorsque ce dernier est immergé dans l'eau. Pour ce faire, il faut calculer la limite de réfraction du rayon incident.

Etant donné que l'eau possède un indice de réfraction inférieur à celui du prisme, plus l'angle d'incidence du rayon lumineux augmente, plus le rayon réfracté dans l'eau s'écarte de la normale à la face convergente. Ainsi, la limite de réfraction est atteinte lorsque le rayon réfracté rase la surface de la première face convergente du prisme, c'est-à-dire que l'angle i2 est égal à 90° par rapport à la normale de la première face convergente du prisme.

Ainsi, en application de la loi de Snell-Descartes :

$$n1 \times \sin(i1) = n2 \times \sin(i2)$$

$$n1 \times \sin(i1) = 1.33 \times \sin(90°)$$

$$n1 \times \sin(i1) = 1.33$$

Lorsque le prisme est conçu de sorte que: $n1 \times \sin(i1) = 1.33$, le rayon lumineux incident est réfracté à la limite de réfraction, il rase la première face convergente du prisme. Cela signifie que lorsque le prisme est conçu de sorte que : $n1 \times \sin(i1) > 1.33$ le rayon incident n'est plus réfracté. Le rayon incident dépasse la limite de réfraction et est totalement réfléchi par la face première convergente. Le rayon incident est ensuite réfléchi sur la deuxième face convergente et est renvoyé vers les moyens de calcul et de traitement par l'intermédiaire de la fibre optique, ce qui permet de détecter la présence d'eau.

Par construction, l'angle de convergence des faces convergentes du prisme est égal à l'angle d'incidence du rayon lumineux par rapport à la normale de la première face convergente du prisme. Ainsi, pour réaliser l'invention, il convient d'ajuster l'angle de convergence des faces convergentes par rapport à la face principale du prisme, en fonction de l'indice de réfraction dudit prisme.

me.

Le principe de l'invention consiste à détecter de l'eau par l'intermédiaire de la réflexion totale du rayon incident. En pratique, la connexion entre la fibre optique et le prisme n'est jamais totalement orthogonale de sorte que la détection d'eau peut se faire en détectant un maximum d'intensité lumineuse réfléchie. Une faible partie du rayon lumineux incident peut encore être réfractée dans l'eau sans nuire à l'invention et sans sortir du cadre de l'invention. L'eau sera tout de même détectée.

De préférence, le prisme est réalisé dans un matériau comprenant un indice de réfraction n1 compris entre 1.33 et 1.40. Selon une forme de réalisation particulière, le prisme est réalisé en Polysulfone dont l'indice de réfraction est environ égal à 1.38. De cette façon, lorsque le prisme en Polysulfone est immergé dans l'eau et en application de la formule de Snell-Descartes on trouve une limite de réfraction pour un angle incident égal à 74.5° :

$$n1 \times \sin(i1) = 1.33 \times \sin(90°)$$

$$1.38 \times \sin(i1) = 1.33$$

$$\sin(i1) = 0964$$

$$i1 = 74.5°$$

Cela signifie que lorsque le prisme en Polysulfone est immergé dans l'eau et que l'angle du rayon incident par rapport à la normale de la première face convergente est supérieur à 74.5°, le rayon incident n'est plus réfracté. Le rayon incident est totalement réfléchi et l'eau est détectée.

De préférence, l'angle du rayon incident par rapport à la normale de la première face convergente est inférieur à 87.5° pour que le rayon incident ne soit pas totalement réfléchi lorsque le prisme en Polysulfone est immergé dans du carburant. Ainsi, l'angle du rayon incident par rapport à la normale de la première face convergente est compris entre 74.5° et 87.5°.

De préférence, le réservoir de carburant comprend une pluralité de capteurs agencés à des niveaux différents à partir du fond du réservoir pour connaitre la quantité d'eau présente dans le fond dudit réservoir, lesdits capteurs étant tous reliés aux mêmes moyens de calcul et de traitement.

Ainsi, l'invention permet de réaliser un multiplexage des capteurs, et d'interroger plusieurs capteurs par l'intermédiaire de moyens de calcul et de traitement uniques, ce qui permet de réduire le poids, l'encombrement et les coûts du système à intégrer dans un aéronef.

L'invention couvre également un réservoir comprenant un système de drainage agencé au fond du réservoir et comportant par exemple une valve actionnable de l'extérieur du réservoir et un ou plusieurs tuyaux permettant d'évacuer l'eau. Selon l'invention, le capteur d'eau est agencé dans ledit système de drainage.

## DESCRIPTION SOMMAIRE DES FIGURES

D'autres avantages et caractéristiques ressortiront mieux de la description qui va suivre, donnée à titre d'exemple non limitatif, du réservoir de carburant selon l'invention, à partir des dessins annexés dans lesquels :

- la figure 1 est une représentation schématique d'un capteur d'eau mis en oeuvre dans le réservoir de carburant selon l'invention ;

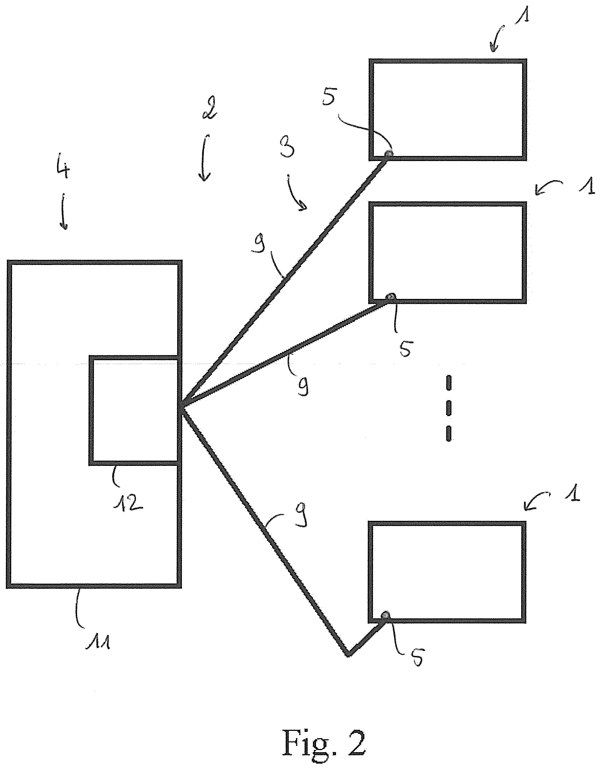

- la figure 2 est une représentation schématique illustrant un système de détection d'eau selon l'invention, mettant en oeuvre un multiplexage de plusieurs capteurs.

## DESCRIPTION DETAILLEE DE L'INVENTION

L'invention concerne un réservoir de carburant (1) d'un aéronef comprenant un dispositif (2) de détection d'eau permettant de fournir des informations sur la nécessité d'effectuer ou non des opérations de drainage pour limiter les immobilisations de l'aéronef et les dépenses inutiles.

Plus précisément, le dispositif (2) de détection comprend un capteur d'eau (3), agencé au fond dudit réservoir (1) et connecté à des moyens de calcul et de traitement (4) pour envoyer un signal de présence d'eau.

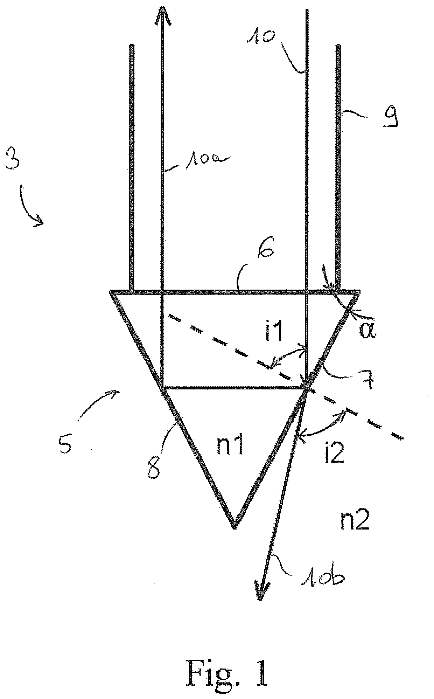

A cet effet, et en référence à la figure 1, le capteur d'eau (3) comprend un prisme (5) à section triangulaire isocèle. Le prisme (5) est par exemple réalisé Polysulfone et comprend un indice de réfraction de 1.38. Le prisme (5) comprend une face principale (6) et deux faces convergentes (7, 8) par rapport à la face principale (6), selon un angle de convergence a.

Une fibre optique (9) est connectée, d'une part, orthogonalement à la face principale (6) du prisme (5) et, d'autre part, aux moyens de calcul et de traitement (4), lesquels comprennent une source lumineuse permettant d'envoyer au moins un rayon lumineux (10) au travers du prisme (5), jusqu'à la première face convergente (7).

En fonction du milieu dans lequel est immergé le prisme (5), le rayon lumineux (10) est destiné à se réfracter, ou à se réfléchir totalement contre la deuxième face convergente (8) qui renvoie elle-même le rayon lumineux (10) au travers de la fibre optique (9) jusqu'aux moyens de calcul et de traitement (4).

Le prisme (5) est conçu de sorte à réfléchir totalement le rayon incident et renvoyer le rayon réfléchi (10a) dans la même direction que le rayon (10) incident lorsqu'il est immergé dans de l'eau, et à transmettre et réfracter le rayon incident (10) lorsque le prisme (5) est immergé dans du carburant. Le rayon réfracté est référencé (10b) sur la figure 1.

En effet, l'angle de convergence α des faces convergentes (7, 8) du prisme (5) est compris entre 74.5° et 87.5°. Par construction, l'angle d'incidence il du rayon lumineux (10) par rapport à la normale de la première face convergente du prisme (5) est égal à l'angle de convergence α et est donc aussi compris entre 74.5° et 87.5°. Cet angle correspond à la limite de réfraction calculée en application de la loi de Snell-Descartes de sorte que pour un angle de convergences a, et donc pour un angle d'incidence il du rayon lumineux (10) compris entre 74.5° et 87.5°, le rayon incident (10) n'est plus réfracté dans l'eau mais est totalement réfléchi vers la deuxième face convergente (8) et renvoyé vers les moyens de calcul et de traitement (4) qui détectent ainsi la présence de l'eau, et le rayon incident est toujours réfracté dans le carburant et n'est jamais réfléchi.

Les moyens de calcul et de traitement (4) sont de tout type approprié pour permettre de recevoir un signal de présence d'eau envoyé par ledit capteur (3), et renvoyer une information sur la nécessité d'effectuer ou non une opération de drainage du réservoir de carburant. Les moyens de calcul et de traitement comprennent par exemple un photo-détecteur pour déterminer lorsque le rayon lumineux (10) et renvoyé. Les moyens de calcul et de traitement (4) comprennent un calculateur (11) et un interrogateur (12) pour interroger le capteur (3) sur la présence d'eau par l'émission d'un rayon lumineux (10). Les moyens de calcul et de traitement (4) sont ensuite aptes à interpréter cette information de présence d'eau afin de fournir une information sur la nécessité d'effectuer ou non une opération de drainage en fonction de la quantité d'eau présente dans le fond du réservoir (1). Par exemple, l'opération de drainage peut être recommandée lorsque l'eau dépasse un certain seuil.

Avantageusement, le réservoir de carburant (1) comprend une pluralité de capteurs (3) agencés à des niveaux différents à partir du fond du réservoir (1). Les différents capteurs (3) sont tous reliés aux mêmes moyens de calcul et de traitement (4). En effet, les fibres optiques (9) permettent de réaliser un multiplexage de la pluralité des prismes (5). Les moyens de calcul et de traitement (4) permettent alors d'interroger les différents capteurs (3) et en fonction de leur réponse, une information sur la hauteur de l'eau présente dans le réservoir (1) peut être donnée. Cette information permet notamment de calculer la quantité d'eau présente dans le réservoir (1).

Les prismes (5) peuvent également être directement agencés dans le fond du réservoir (1), par exemple dans un système de drainage que comprend le réservoir (1). Le système de drainage agencé au fond du réservoir comprend par exemple une valve actionnable de l'extérieur du réservoir et un ou plusieurs tuyaux permettant d'évacuer l'eau. Les prismes (5) peuvent être agencés dans la valve ou dans les tuyaux.

Les dispositifs de détection d'eau (2) décrits peuvent être montés dans tous les réservoirs (1) de l'aéronef de sorte que tous les prismes (5) sont multiplexés et reliés par l'intermédiaire des fibres optiques (9) aux mêmes moyens de calcul et de traitement (4), ce qui permet de réduire le poids, l'encombrement et les coûts du système à intégrer dans un aéronef.

Il ressort de ce qui précède le réservoir (1) selon l'invention comprend un dispositif (2) de détection d'eau qui permet de fournir des informations sur la nécessité d'effectuer des opérations de drainage pour limiter les immobilisations de l'aéronef et les dépenses inutiles

**Revendications**

1. Réservoir de carburant (1) d'un aéronef comprenant un capteur d'eau (3), agencé au fond dudit réservoir (1) et comprenant un élément optique (9) apte à permettre de différencier l'eau du carburant connecté à des moyens de calcul et de traitement (4) pour recevoir un signal de présence d'eau envoyé par ledit capteur (3), *caractérisé en ce que* les moyens de calcul et de traitement (4) sont configurés pour interpréter le signal de présence d'eau et fournir une information sur la nécessité d'effectuer ou non une opération de drainage en fonction de la quantité d'eau présente dans le fond du réservoir (1), et l'élément optique (9) est connecté aux moyens de calcul et de traitement (4) par l'intermédiaire d'au moins une fibre optique (9).

2. Réservoir de carburant (1) selon la revendication 1, *caractérisé en ce que* la fibre optique (9) est connectée, d'une part, à l'élément optique et, d'autre part, à une source lumineuse pour envoyer au moins un rayon lumineux (10) vers ledit élément optique, ledit élément optique étant apte :

   - à réfléchir et à renvoyer le rayon lumineux (10) dans la fibre optique (9) vers les moyens de calcul et de traitement (4) lorsque ledit élément optique est immergé dans de l'eau, et ;
   - à réfracter et à ne pas renvoyer le rayon lumineux (10) lorsque ledit élément optique est immergé dans du carburant.

3. Réservoir de carburant (1) selon la revendication 2, *caractérisé en ce que* l'élément optique se présente sous la forme d'un prisme (5) à section triangulaire isocèle de manière à définir au moins une face principale (6) et deux faces convergentes (7, 8), ledit prisme (5) étant réalisé dans un matériau transparent à la lumière comprenant un indice de réfraction égal à n1, avec n1 supérieur à 1.33, la fibre optique (9) est connectée orthogonalement à la face principale (6) du prisme (5) de sorte que le rayon lumineux (10) incident est destiné à se réfracter et/ou se réfléchir sur une première face convergente avec un angle il par rapport à la normale de la première face convergente, ledit prisme (5) étant conçu de sorte que : $n1 \times sin(i1) > 1.33$

4. Réservoir de carburant (1) selon la revendication 3, *caractérisé en ce que* l'indice de réfraction n1 du prisme (5) est inférieur à 1.40.

5. Réservoir de carburant (1) selon la revendication 4, *caractérisé en ce que* le prisme (5) est réalisé en Polysulfone et comprend un indice de réfraction de 1.38, l'angle de convergence $\alpha$ entre les faces convergentes (7, 8) et la face principale (6) est compris entre 74.5° et 87.5°.

6. Réservoir de carburant (1) selon l'une des revendications 1 à 5, *caractérisé en ce qu'*il comprend une pluralité de capteurs (3) agencés à des niveaux différents à partir du fond du réservoir (1) pour connaitre la quantité d'eau présente dans le fond dudit réservoir (1), lesdits capteurs (3) étant tous reliés aux mêmes moyens de calcul et de traitement (4).

7. Réservoir de carburant (1) selon l'une des revendications 1 à 6, *caractérisé en ce qu'*il comprend un système de drainage agencé au fond du réservoir (1), le capteur d'eau (3) étant agencé dans ledit système de drainage.

**Patentansprüche**

1. Treibstofftank (1) eines Luftfahrzeugs umfassend einen Wassersensor (3), der am Boden dieses Tanks (1) angeordnet ist und der ein optisches Element (9) enthält, mit dem Wasser vom Treibstoff unterschieden werden kann, der mit Berechnungs- und Verarbeitungsvorrichtungen (4) verbunden ist, um ein Signal von diesem Sensor (3) zu empfangen, ob Wasser vorhanden ist, *dadurch gekennzeichnet dass* die Berechnungs- und Verarbeitungsvorrichtungen (4) konfiguriert sind, um das Signal für vorhandenes Wasser zu interpretieren und eine Information darüber zu geben, ob ein Drainagevorgang in Abhängigkeit von der Wassermenge durchzuführen ist oder nicht, die sich am Tankboden (1) angesammelt hat und dass das optische Element (9), ist mit den Berechnungs- und Verarbeitungsvorrichtungen (4) über mindestens eine optische Faser (9) verbunden.

2. Treibstofftank (1) eines Luftfahrzeugs gemäß Anspruch 1, *dadurch gekennzeichnet,* dass die optische Faser (9), einerseits mit dem optischen Element und andererseits mit einer Leuchtquelle verbunden ist, um mindestens einen Lichtstrahl (10) zu diesem optischen Element zu strahlen, wobei dieses optische Element in der Lage ist:

- den Lichtstrahl (10) zu reflektieren und in die optische Faser (9) zu den Berechnungs- und Verarbeitungsvorrichtungen (4)zurückzuleiten, wenn dieses optische Element in Wasser eingetaucht ist, und

- den Lichtstrahl (10) zu brechen und nicht zurückzustrahlen, wenn dieses optische Element in Treibstoff eingetaucht ist.

3. Treibstofftank (1) eines Luftfahrzeugs gemäß Anspruch 2, **dadurch gekennzeichnet, dass** das optische Element die Form eines Prismas (5) mit dem Querschnitt eines gleichschenkligen Dreiecks hat, so dass mindestens eine Hauptseite (6) und zwei konvergierende Seiten (7, 8) definiert sind, wobei dieses Prisma (5) aus einem lichtdurchlässigen Material ausgeführt ist, dessen Brechungsindex gleich n1 ist, mit n1 größer 1.33, wobei die optische Faser (9) orthogonal mit der Hauptseite (6) des Prismas (5) verbunden ist, so dass der einfallende Lichtstrahl (10) gebrochen und/ oder mit einem Winkel i1 bezogen auf die Normale der ersten konvergierenden Seite auf eine erste konvergierende Fläche reflektiert wird, wobei dieses Prisma (5) so konzipiert ist, dass n X sin (i1) > 1.33 ist.

4. Treibstofftank (1) eines Luftfahrzeugs gemäß Anspruch 3, **dadurch gekennzeichnet, dass** der Brechungsindex n1 des Prismas (5) kleiner ist als 1.40.

5. Treibstofftank (1) eines Luftfahrzeugs gemäß Anspruch 4, **dadurch gekennzeichnet, dass** das Prisma (5) aus Polysulfon besteht und einen Brechungsindex von 1.38 hat, wobei der Konvergenzwinkel $\alpha$ zwischen den konvergierenden Seiten (7, 8) und der Hauptseite (6) zwischen 74.5° und 87.5° liegt.

6. Treibstofftank (1) eines Luftfahrzeugs gemäß einem der Ansprüche 1 bis 5, **dadurch gekennzeichnet, dass** es mehrere Sensoren (3) enthält, die auf verschiedenen Höhen vom Boden des Treibstofftanks (1) angeordnet sind, um zu wissen, wieviel Wasser auf dem Boden dieses Treibstofftanks (1) vorhanden ist, wobei diese Sensoren (3) alle mit denselben Berechnungs- und Verarbeitungsvorrichtungen (4) verbunden sind.

7. Treibstofftank (1) eines Luftfahrzeugs gemäß einem der Ansprüche 1 bis 6, **dadurch gekennzeichnet, dass** er ein Drainagesystem, das am Boden dieses Treibstofftanks (1) angeordnet ist, enthält, wobei der Wassersensor (3) in diesem Drainagesystem angeordnet ist.

**Claims**

1. An aircraft fuel tank (1) comprising a water sensor (3), arranged at the bottom of said tank (1) and comprising an optical component (9) being able to differentiate between water and fuel connected to calculation and processing means (4) in order to receive a presence of water signal sent by said sensor (3), **characterized** in that calculation and processing means (4) are configured to interpret the water presence signal and provide information on whether or not it is necessary to carry out a drainage operation depending on the quantity of water present in the bottom of the tank (1), and the optical component (9) is connected to the calculation and treatment means (4) via at least one optical fiber (9).

2. The fuel tank (1) according to claim 1, **characterized in that** the optical fiber (9) is connected, on the one hand, to the optical component and, on the other hand, to a light source in order to send at least one light beam (10) to said optical component, said optical component being suitable:

- to reflect and return the light beam (10) within the optical fiber (9) to the calculation and processing means (4) when said optical component is immersed in water, and;
- to refract and not return the light beam (10) when said optical component is immersed in fuel.

3. The fuel tank (1) according to claim 2, **characterized in that** the optical component is in the form of a prism (5) with an isosceles triangular section in such a manner as to define at least one main side (6) and two convergent sides (7, 8), said prism (5) being made of a material that is transparent to light with a refractive index equal to n1, wherein n1 is greater than 1.33, the optical fiber (9) is connected orthogonally to the main side (6) of the prism (5) such that the incident light beam (10) is intended to refract and/or reflect on a main convergent side with an angle i1 relative to the normal of the first convergent side, said prism (5) being designed such that: $n1 \times sin (i1) > 1.33$

4. The fuel tank (1) according to claim 3, **characterized in that** the refractive index of the prism (5) n1 is less than 1.40.

5. The fuel tank (1) according to claim 4, **characterized in that** the prism (5) is made of Polysulfone and comprises a refractive index of 1.38, the convergence angle $\alpha$ between the convergent sides (7, 8) and the main side (6) is between 74.5° and 87.5°.

6. The fuel tank (1) according to any of claims 1 to 5, **characterized in that** it comprises a plurality of sensors (3) arranged at different levels from the bottom of the tank (1) in order to know the amount of water present at the bottom of said tank (1), all of said sensors (3) being connected to the same calculation and processing means (4).

7. The fuel tank (1) according to any of claims 1 to 6, *characterized* **in that** it comprises a drainage system arranged at the bottom of the tank (1), the water sensor (3) being arranged within said drainage system.

Fig. 1

Fig. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0795740 A1